(19) Europäisches Patentamt
European Patent Office
Office européen des brevets

(11) Veröffentlichungsnummer: **0 083 729**
**B1**

(12) # EUROPÄISCHE PATENTSCHRIFT

(45) Veröffentlichungstag der Patentschrift: **26.02.86**

(51) Int. Cl.⁴: **C 07 D 233/50**, A 61 K 31/415

(21) Anmeldenummer: **82111148.1**

(22) Anmeldetag: **02.12.82**

(54) **Substituierte 1-Benzoyl-2-phenylimino-imidazolidine, deren Säureadditionssalze, Verfahren zu deren Herstellung und diese enthaltende Arzneimittel.**

(30) Priorität: **07.01.82 DE 3200258**

(43) Veröffentlichungstag der Anmeldung: **20.07.83 Patentblatt 83/29**

(45) Bekanntmachung des Hinweises auf die Patenterteilung: **26.02.86 Patentblatt 86/9**

(84) Benannte Vertragsstaaten: **AT BE CH DE FR GB IT LI LU NL SE**

(56) Entgegenhaltungen:
**BE - A - 741 947**
**DE - A - 2 542 702**
**US - A - 4 262 005**

(73) Patentinhaber: **CHEMIE LINZ AKTIENGESELLSCHAFT, St. Peter-Strasse 25, A-4020 Linz (AT)**
(84) Benannte Vertragsstaaten: **BE CH FR GB IT LI LU NL SE AT**

(73) Patentinhaber: **Lentia Gesellschaft mit beschränkter Haftung, Arabellastrasse 4 Postfach 81 05 08, D-8000 München 81 (DE)**
(84) Benannte Vertragsstaaten: **DE**

(72) Erfinder: **Franzmair, Rudolf, Dr., Franz-Xaver-Müllerweg 9, A-4033 Linz (AT)**
Erfinder: **Enzenhofer, Rita, Dr., Stifterstrasse 12, AT-4100 Ottensheim (AT)**

**EP 0 083 729 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue substituierte 1-Benzoyl-2-phenyl-imino-imidazolidine, deren Säureadditionssalze, Verfahren zu deren Herstellung sowie Arzneimittel, welche die neuen Verbindungen als Wirkstoff enthalten, besonders solche mit analgetischer Wirkung.

Aus der deutschen Offenlegungsschrift Nr. 2811847 ist bekannt, daß 1-Cycloalkenoyl-2-phenylimino-imidazolidine, beispielsweise 1-(Cyclohex-3''-en-1''-oyl)-2-(2',6'-dichlorphenylimino)-imidazolidin, analgetische Eigenschaften aufweisen und als Wirkstoffe in pharmazeutischen Präparaten zur Behandlung von Schmerzzuständen verwendet werden können.

Andererseits sind in der DE—PS 2559711 Benclonidin (1-Benzoyl-2-(2',6'-dichlorphenylimino)-imidazolidin) sowie die drei isomeren Toluylderivate des Clonidin als Verbindungen beschreiben, die sich in erster Linie durch eine ausgeprägt antihypertensive Wirkung auszeichnen und zur Herstellung von Lösungen für Injektionszwecke und insbesondere von peroral zu verabreichenden Arzneimitteln zur Behandlung der Hypertonie geeignet sind.

Schließlich sind in der DE—OS 3011327 solche 1-Aroyl-2-phenylimino-imidazolidine geoffenbart, bei denen der Aroylrest einen gegegebenenfalls substituierten mono- oder bicyclischen heteroaromatischen Acylrest darstellt. Diese Verbindungen sollen ebenfalls stark blutdrucksenkend wirken.

Es wurden nun neue 1-Benzoyl-2-phenylimino-imidazolidinderivate gefunden, welche am Benzoylrest mindestens einen Substituenten mit überwiegend elektronegativen Eigenschaften tragen. Überraschenderweise haben diese neuen Verbindungen eine ausgeprägt analgetische Wirkung, während antihypertensive und zentraldämpfende Eigenschaften nur in ganz geringem Ausmaß beobachtet werden.

Gegenstand der Erfindung sind substituierte 1-Benzoyl-2-phenylimino-imidazolidine der allgemeinen Formel

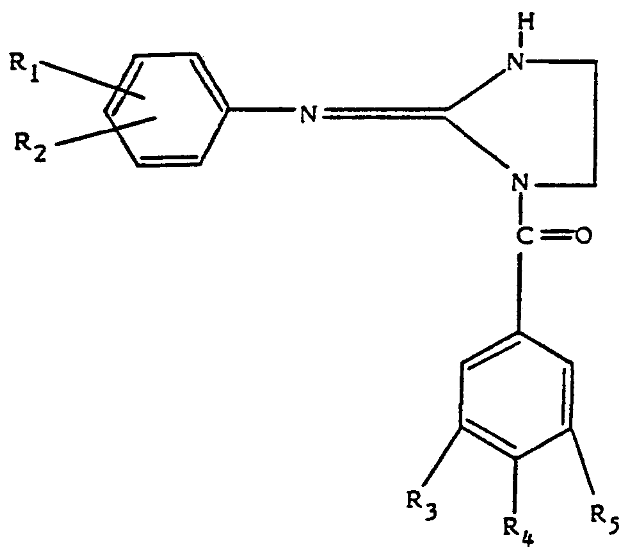

I,

in der

$R_1$ und $R_2$ unabhängig voneinander Wasserstoff, Halogen, die Methyl- oder Methoxygruppe bedeuten und

$R_3$, $R_4$ und $R_5$ unabhängig voneinander für Wasserstoff, die Methylgruppe oder für elektronegative Substituenten aus der Gruppe Halogen, geradkettiger oder verzweigter $C_1$ bis $C_4$-Alkoxyrest, Cyano-, Nitro- oder Methylsulfonlyrest stehen, mit der Maßgabe, daß einer der Reste $R_3$ bis $R_5$ immer ein elektronegativer Substituent aus der Gruppe Halogen, geradkettiger oder verzweigter $C_1$ bis $C_4$-Alkoxyrest, Cyano-, Nitro- oder Methylsulfonylrest ist und die beiden anderen Reste unabhängig voneinander die für $R_3$, $R_4$ bzw. $R_5$ angegebene Bedeutung haben, sowie die pharmazeutisch annehmbaren Säureadditionssalze dieser Verbindung, wobei solche Verbindungen der Formel I ausgenommen sind, bei denen $R_1$ 2-Chlor und $R_2$ 4-Chlor bedeutet.

Vorteilhafte Bedeutungen für $R_1$ und $R_2$ sind Chlor, Brom, Fluor, Methyl oder Methoxy. Bevorzugt ist ferner, daß $R_1$ und $R_2$ gleichzeitig Halogen bedeuten und einer der Reste $R_1$ oder $R_2$ in ortho-Stellung am Phenylrest steht. Besonders bevorzugt sind 2,6-Dichlor-, 2,3-Dichlor oder, 2,6-Dibromphenyliminoimidazolidine sowie 2-Chlor-4-methyl-, 2-Chlor-6-methyl, 2,6-Dimethyl- oder auch 2-Methoxyphenyliminoimidazolidine der allgemeinen Formel I.

Bevorzugte Verbindungen innerhalb der Formel I sind weiters solche, in denen $R_3$, $R_4$ oder $R_5$ die Cyano-, Nitro-, Methylsulfonylgruppe, einen geradkettigen oder verzweigten $C_1$ bis $C_4$-Alkoxyrest Chlor oder Fluor bedeuten. Ganz besonders bevorzugte Verbindungen sind solche, bei denen einer der Reste $R_3$, $R_4$ oder $R_5$ der Cyano-, Fluor-, Nitro-, Methylsulfonyl-, Chlor- oder Methoxyrest ist und die beiden anderen Reste unabhängig voneinander Wasserstoff oder die Methylgruppe bedeuten.

Gegenstand der Erfindung ist weiters ein Verfahren zur Herstellung der Verbindungen der Formel I, welches darin besteht, daß man

a) ein Anilinderivat der allgemeinen Formel

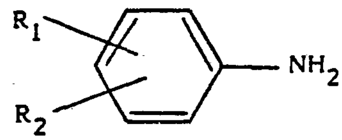

II,

worin $R_1$ und $R_2$ die oben angeführte Bedeutung besitzen, mit einem substituierten 1-Benzoyl-imidazolidin-2-on der allgemeinen Formel

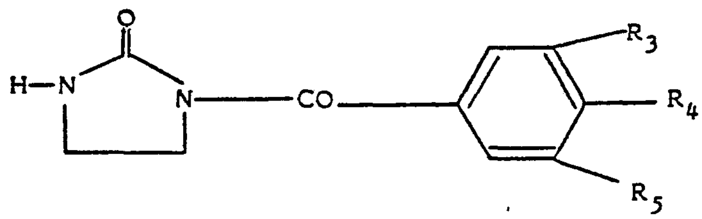

III,

worin $R_3$ bis $R_5$ wie in Formel I definiert sind, im Molverhältnis 1:1 bis 1:1,1 in Gegenwart von mindestens 2 Moläquivalenten Phosphoroxychlorid umsetzt oder

b) ein substituiertes 2-Phenylimino-imidazolidin der allgemeinen Formel

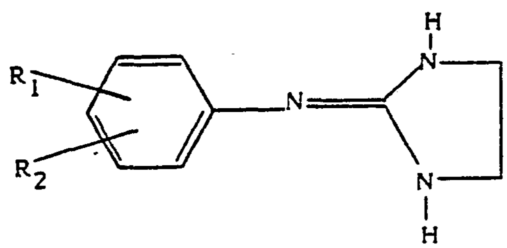

IV,

worin $R_1$ und $R_2$ die oben angeführte Bedeutung besitzen, mit einem aktiven Amid der allgemeinen Formel

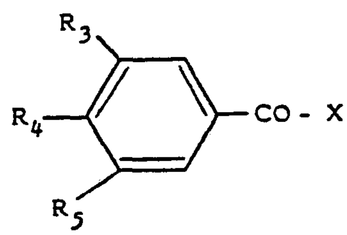

V,

umsetzt, in der $R_3$ bis $R_5$ wie in Formel I definiert sind und X den Rest eines quasiaromatischen fünfgliederigen Heterocyclus mit mindestens 2 Stickstoffatomen im Ring, der gegebenenfalls mit einem Benzolkern kondensiert sein kann, oder den Rest der allgemeinen Formel

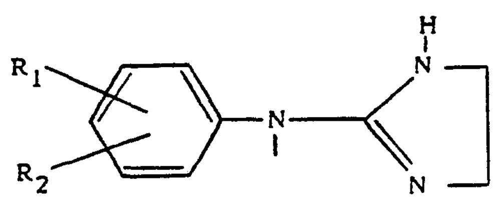

VI,

darstellt, wobei $R_1$ und $R_2$ die in Formel I angegebene Bedeutung besitzen oder

c) ein substituiertes N-Benzoyläthylendiamin der allgemeinen Formel

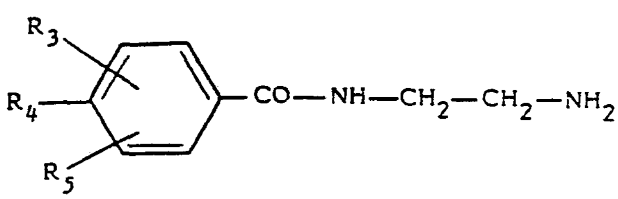

VII,

in der $R_3$, $R_4$ und $R_5$ die oben angegebene Bedeutung besitzen, mit einem Isocyaniddichlorid der allgemeinen Formel

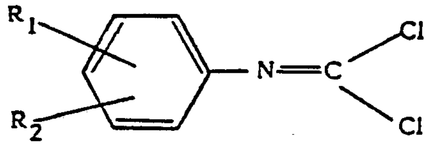

VIII,

in der $R_1$ und $R_2$ die oben angegebene Bedeutung besitzen in Gegenwart von 2 Moläquivalenten eines Alkalihydroxids oder Alkalihydrids als säurebindendes Mittel umsetzt und die so erhaltenen Verbindungen gegebenenfalls mit Säuren in ihre pharmazeutisch annehmbaren Salze überführt.

Die Umsetzung des Amins der allgemeinen Formel II gemäß a) mit einem substituierten 1-Benzoyl-2-imidazolidin-2-on erfolgt zweckmäßig bei Temperaturen zwischen Raumtemperatur und der Siedetemperatur des Phosphoroxychlorids, bevorzugt aber bei Temperaturen zwischen 50°C und 80°C. Lösungsmittel sind nicht erforderlich, können aber verwendet werden. Als solche kommen vor allem inerte organische, besonders Chlorkohlenwasserstoffe wie Methylenchlorid und Chloroform, in Frage. Besonders günstig ist es, die Umsetzung in überschüssigem Phosphoroxychlorid als Lösungsmittel auszuführen. Die Reaktionszeit richtet sich sowohl nach der Temperatur, bei der die Umsetzung erfolgt, als auch nach der Reaktivität der eingesetzten Komponenten und schwankt zwischen einigen Stunden und mehreren Tagen.

Zur Aufarbeitung wird das Lösungsmittel bzw. das überschüssige Phosphoroxychlorid im Vakuum möglichst vollständig entfernt und der harzige Rückstand, der ein Phosphoroxychloridadditionsprodukt der erfindungsgemäßen Verbindung der allgemeinen Formel I darstellt, in einem mit Wasser nicht mischbaren Solvens aufgenommen und durch milde Hydrolyse in Verbindungen der Formel I übergeführt. Die milde Hydrolyse wird in der Regel unter Eiskühlung durch langsames Zutropfen basischer Reagenzien, beispielsweise Natriumcarbonatlösung oder verdünnter Natronlauge, ausgeführt, wobei die Hydrolyse sowohl durch direkte Zugabe des Hydrolysemittels zum Eindampfrückstand als auch durch Auflösen derselben in einem organischen Lösungsmittel und Behandeln der Lösung mit dem alkalischen Hydrolysemittel erfolgen kann.

Eine besonders günstige Ausführungsform liegt dann vor, wenn das Phosphoroxychloridaddukt der Verbindung der allgemeinen Formel I durch Zusatz eines geeigneten Lösungsmittels in kristalliner Form gewonnen werden kann. In diesem Fall wird das kristalline Produkt in Eiswasser suspendiert und wie oben beschreiben, der milden Hydrolyse unterworfen. Die erfindungsgemäßen Verbindungen der allgemeinen Formel I fallen dabei kristallin und in ausgezeichneter Reinheit an.

Die als Ausgangsprodukte verwendeten 1-Benzoyl-imidazolidin-2-one der allgemeinen Formel III sind teilweise noch nicht beschreiben. Sie lassen sich durch Umsetzung von 2 Moläquivalenten Imidazolidin-2-on mit dem entsprechend substituierten Benzoylchlorid in einem polaren Lösungsmittel, beispielsweise Acetonitril, in bekannter Weise erhalten.

So kann beispielsweise zur Herstellung von 1-(p-Methoxybenzoyl)-imidazolidin-2-on wie folgt verfahren werden. 34,4 g (0,4 Mol) wasserfreies und fein verriebenes Imidazolidin-2-on werden in 300 ml wasserfreiem Acetonitril suspendiert und unter Rühren mit 34,1 g (0,2 Mol) p-Methoxybenzoylchlorid, welches vorher in 70 ml wasserfreiem Acetonitril gelöst wurde, tropfenweise versetzt. Zur Vervollständigung der Reaktion wird noch 20 Stunden bei Raumtemperatur gerührt. Anschließend wird der Trockenrückstand hergestellt, der in Wasser digeriert, abfiltriert, mit Wasser gewaschen und getrocknet wird. Nach dem Umkristallisieren aus Isopropanol erhält man 25,17 g (57,2 % der Th) 1-(p-Methoxybenzoyl)-imidazolidin-2-on vom Fp. 173—176°C.

In anolger Weise wurden die folgenden Verbindungen erhalten:

1-(p-Nitrobenzoyl)-imidazolidin-2-on (78,2 % aus 90 % Acetonitril,) Fp. 229—240°C (Zersetzung)
1-(m-Fluorbenzoyl)-imidazolidin-2-on (73,2 % aus Toluol), Fp. 160—163°C
1-(p-Fluorbenzoyl)-imidazolidin-2-on (77,9 % aus Toluol,) Fp. 159—161°C
1-(p-Chlorbenzoyl)-imidazolidin-2-on (69,3 % aus 80 % Acetonitril), Fp. 187—192°C
1-(3,4,5-Trimethoxybenzoyl)-imidazolidin-2-on (60,3 % aus Isopropanol), Fp. 174—176°C
1-(3,4-Dimethoxybenzoyl)-imidazolidin-2-on (80 % aus Acetonitril), Fp. 181—183°C
1-(3,5-Dimethoxybenzoyl)-imidazolidin-2-on (82,9 % aus Isopropanol), Fp. 183—186°C
1-(p-Cyanobenzoyl)-imidazolidin-2-on (91 % aus Isopropanol), Fp. 212—216°C
2-(p-n-Propoxybenzoyl)-imidazolidin-2-on (76,5 % aus Acetonitril), Fp. 185—189°C

Für die Umsetzung b) kommen als aktive Amide der allgemeinen Formel V vor allem die Azolide in Frage, wobei der Rest X in Formel V beispielsweise den Rest eines Imidazol, Pyrazol, 1,2,3-Triazol, 1,2,4-Triazol, Tetrazol, Benzimidazol, Bentriazol oder deren Substitutionsprodukte sein kann.

Die Azolide der allgemeinen Formel V können nach bekannten Methoden aus dem entsprechend substituierten Benzoylchlorid und 2 Äquivalenten einer der oben genannten heterocyclischen Verbindungen mit mindestens 2 Stickstoffatomen im Ring hergestellt werden (vgl. dazu die Arbeit von H.A. Staab und W. Rohr "Synthesen mit heterocyclischen Amiden" erschienen in W. Foerst, Neuere Methoden der präparativen organischen Chemie, B and V, Seite 33 ff). Von den Azoliden sind die Imidazolide aufgrund ihrer leichten Zugänglichkeit besonders bevorzugt. Sie können entweder nach der oben angegebenen

Methode oder durch Umsetzung des entsprechend substituierten Benzoesäure mit einem Äquivalent N,N'-Carbonyldiimidazol in einem inerten Lösungsmittel hergestellt werden.

Es ist aber nicht in allen Fällen notwendig, die Azolide der Formel V in einem getrennten Arbeitsgang herzustellen. Sie können bevorzugt in situ aus einem der genannten Azole und dem entsprechenden Säurechlorid hergestellt und direkt weiterverwendet werden. Bei dieser Verfahrensvariante wird das Acylchlorid beispielsweise in Tetrahydrofuran mit dem Azol umgesetzt, vom entstandenen Azolhydrochlorid abfiltriert und das 2-Phenylimino-imidazolidin der allgemeinen Formel VI direkt in die Lösung des Azolids eingetragen.

Zweckmäßig wird so verfahren, daß man das substituierte 2-Phenylimino-imidazolidin mit einem 10 % Überschuß an Azolid bei Temperaturen zwischen Raumtemperatur und der Siedetemperatur des Lösungsmittels in Abhängigkeit von der Reaktivität der Reste X im Azolid umsetzt. Als Lösungsmittel Können aprotische polare und apolare Solventien verwendet werden. Als solche Lösungsmittel kommen beispielsweise aromatische Kohlenwasserstoffe wie Benzol, Xylol oder Toluol, Äther wie Tetrahydrofuran, Diäthyläther oder Dioxan, Ester wie Äthylacetat, halogenierte Kohlenwasserstoffe wie Methylenchlorid, Chloroform oder Tetrachlorkohlenstoff, Ketone wie Aceton oder Methyläthylketon sowie aprotische polare Lösungsmittel wie Acetonitril, Dimethylformamid oder Dimethylsulfoxid in Betracht. Die Reaktionszeit richtet sich nach der Reaktivität der eingesetzten Komponenten und der gewählten Reaktionstemperatur und beträgt von einigen Stunden bis zu mehreren Tagen.

Eine gunstige Variante des Verfahrens besteht darin, daß man als aktives Amid der allgemeinen Formel V eine Verbindung wählt, bei der X die in der Formel VI angegebene Bedeutung hat. Besonders bevorzugt ist diese Verfahrensvariante dann, wenn die Reste $R_1$ und $R_2$ in der zu acylierenden Verbindung der Formel IV und im Rest X der Formel VI die gleiche Bedeutung haben und die gleiche Stellung am Benzolkern einnehmen. Bei dieser Ausführungsform wird das 2-Phenylimino-imidazolidin der allgemeinen Formel IV mit einem wesentlichen Unterschuß, zweckmäßig unter einem halben Äquivalent, bezogen auf das aktive Amid der Formel V eingesetzt, da ja im Verlaufe der Acylierung die Verbindung der Formel IV kontinuierlich aus dem aktiven Amid der Formel V in Freiheit gesetzt wird. Diese Variante des Verfahrens zur Herstellung der erfindungsgemäßen Verbindungen wird bevorzugt durch Kochen der Ausgangsmateialien in einem inerten aprotischen Lösungsmittel, beispielsweise Toluol oder Xylol durchgeführt.

Aktive Amide, in denen X den Rest der Formel VI darstellt, sind gemäß der belgischen Patentschrift Nr. 741947 durch Umsetzung eines entsprechend substuierten 2-Phenylamino-imidazolidin der Formel IV mit einem entsprechend substituierten Benzoylchlorid zugänglich.

Die Aufarbeitung gestaltet sich bei allen Verfahrensvarianten sehr einfach. Der nach Abdampfen des Lösungsmittels verbleibende Rückstand wird in einem geeigneten Solvens, bevorzugt in Wasser, digeriert, wobei meistens Kristallisation eintritt. Das Rohprodukt wird aus einem geeigneten Solvens umkristallisiert.

Die Umsetzung des N-Benzoyläthylendiamin der Formel VII gemäß c) mit einem Isocyaniddichlorid der Formel VIII erfolgt zweckmäßigerweise bei Anfangstemperaturen von 0°C bis Raumtemperatur, wobei zur Vervollständigung der Reaktion die Temperatur allmählich auf 50°C gesteigert werden kann. Die Reaktion kann sowohl homogen als auch heterogen geführt werden. Bei homogener Reaktionsführung wird entweder in wässerigem Milieu unter Zusatz eines wasserlöslichen organischen Solvens wie beispielsweise Dioxan oder wasserfrei in einem aprotischen Lösungsmittel wie beispielsweise Tetrahydrofuran gearbeitet. Bei heterogener Reaktionsführung wird nach Art einer zweiphasigen Schotten-Baumann-Reaktion in wässeriger Lösung unter Zusatz eines mit Wasser nicht mischbaren organischen Lösungsmittels, beispielsweise Benzol oder Toluol gearbeitet.

Je nach Ausführungsform können als saurebindende Mittel Alkalihydroxide wie Natronlauge oder Kalilauge, bei wasserfreiem Arbeiten auch Alkalihydride wie Natriumhydrid eingesetzt werden. Die Reaktionszeit richtet sich nach der Reaktivität der eingesetzten Komponenten und beträgt zwischen Stunden und einem Tag.

Zur Aufarbeitung wird die organische Phase abgetrennt, neutral gewaschen und getrocknet. Das nach Abdampfen des Lösungsmittels verbleibende Rohprodukt wird schließlich durch Umkristallisieren gereinigt.

Die als Ausgangsverbindungen verwendeten substituierten N-Benzoyläthylendiamine der allgemeinen Formel VII lassen sich nach Aspinal, J. Org. Chem. 6, 895—899 (1941) in üblicher Weise und in guten Ausbeuten erhalten.

Nach der angegebenen Methode wurden beispielsweise fertiggestellt:

N-(p-Chlorbenzoyl)-äthylendiamin (78,7 % d. Th. aus Toluol), Fp. 107—110°C N-(p-Methoxybenzoyl)-äthylendiamin (80,15 % aus Benzol/Äthanol, 2:1), Fp. 110°C—112°C.

Es wird noch darauf hingewiesen, daß die erfindungsgemäßen Verbindungen der allgemeinen Formel I, ebenso wie die 2-Phenylimino-imidazolidine der allgemeinen Formel IV in einem Tautomeriegleichgewicht entsprechend folgender Formeln vorliegen können:

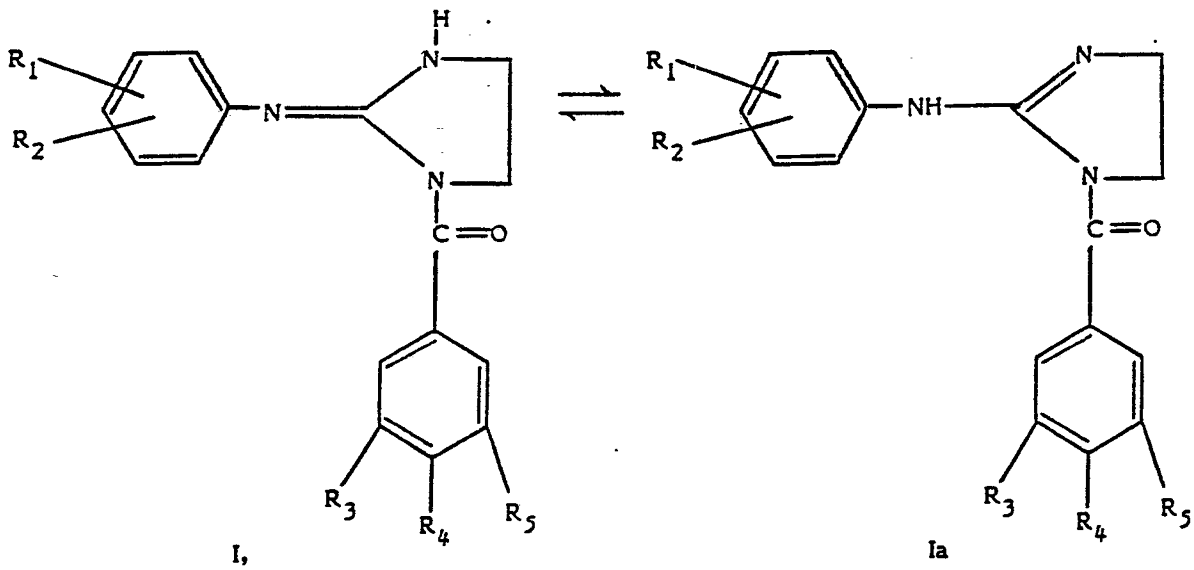

I, Ia

Je nach den Verfahrensbedingungen enthält man das Endprodukt der Formel I bzw. Ia entweder als freie Base oder deren Säureadditionssalze.

Die erfindungsgemäßen basisch reagierenden Verbindungen der allgemeinen Formel I können auf die übliche Weise in ihre physiologisch verträglichen Säureadditionssalze überführt werden. Bei der Herstellung der Säureadditionssalze werden vorzugsweise solche Säuren verwendet, die in J. Pharm. Sci. 66, 1—16 zur Herstellung von therapeutisch verträglichen Salzen beschrieben sind. Zur Salzbildung geeignet sind beispielsweise starke Mineralsäuren wie Chlorwasserstoff-, Bromwasserstoff-, Jodwasserstoff-, Fluorwasserstoffsäure, Schwefelsäure, Phosphorsäure, Salpetersäure und Perchlorsäure, aliphatische, alicyclische, aromatische, heterocyclische Carbonsäuren oder Sulfonsäuren wie Ameisensäure, Essigsäure, Propionsäure, Bernsteinsäure, Glykolsäure, Milchsäure, Apfelsäure, Salicylsäure, Nikotinsäure, Cyclohexylfsulfonsäure, Methansulfonsäure oder Amidosulfonsäuren.

Die neuen Verbindungen der allgemeinen Formel I sowie deren Säureadditionssalze haben wertvolle therapeutische Eigenschaften als Analgetica. Sie sind wegen ihrer Fähigkeit in geringen Dosen bei Säugetieren Schmerzen lindern, als Pharmaka mit stark analgetischen und vergleichsweise geringer oder fehlender antihypertensiver Wirkung zur Behandlung von Schmerzzuständen geeignet. Die analgetischen Eigenschaften der erfindungsgemäßen Verbindungen können u.a. an dem durch Phenyl-2-chinon hervorgerufenen Writhingsyndrom an der Maus, einem Standardtest auf Analgesie (Proc. Soc. Exptl. Biol. Med. 95, 729, 1957), nachgwiesen werden.

Die mit p-Cyanobenzoyl-2-(2',6'-dichlorphenylimino)-imidazolidin im Writhing erzielte $ED_{50}$ beträgt beispielsweise nur 0,55 mg/kg Maus (berechnet nach Thompson und Weil, Biometrics 8, 1952, 51—4, 249—263). Eine blutdrucksenkende Wirkung tritt dabei überraschenderweise überhaupt nicht auf.

Das bekannte Schmerzmittel Pentazocin(R) ist im Vergleich dazu mit einer $ED_{50}$ von 168,18 mg/kg im Writhing wesentlich schwächer analgetisch wirksam.

Gegenstand der vorliegenden Erfindung sind ferner Arzneimittel mit analgetischer Wirksamkeit, welche eine oder mehrere Verbindung der Formel I oder deren Säureadditionssalze als Wirkstoff enthalten. Wirksame Mengen der erfindungsgemäßen Verbindungen können einem Patienten nach irgendeiner von verschiedenen Methoden verabreicht werden, beispielsweise peroral in Form von Tabletten, Kapseln, Dragees, Pulver, Lösungen oder Suspensionen, parenteral in Form steriler Lösungen oder Suspensionen oder intravenös in Form von Lösungen. Die Verbindungen der Formel I sind zwar als freie Basen wirksam, doch kann man sie aus Gründen der Stabilität, Leichtigkeit der Kristallisation, erhöhter Löslichkeit und dergleichen in Form ihrer pharmazeutisch unbedenklichen Additionssalze formulieren und verabreichen. Zur Herstellung von Tabletten, Kapseln, Dragees, Zäpfchen, Pulvern, Lösungen oder Suspensionen können die üblicherweise verwendeten galenischen Hilfs-, Träger-, Spreng-oder Schmiermittel oder Substanzen zur Erzielung einer Depotwirkung Anwendung finden.

Die folgenden Beispiele erläutern die Verbindung.

Beispiel 1

8,10 g (50 mmol) 2,6-Dichloranilin, 11,0 g 1-p-Methoxybenzoyl-imidazolidin-2-on und 75 ml Phosphoroxychlorid werden 70 Stunden lang bei 60°C gerührt. Die entstandene klare gelbe Lösung wird

eingedampft, der Rückstand wird mit Eis versetzt und unter Rühren wird gesättigte Sodalösung zugetropft, bis ein Konstanter pH von etwa 8 erreicht ist.

Dann wird dreimal mit Methylenchlorid extrahiert, die vereinigten Methylenchloridphasen werden mit Wasser gewaschen, getrocknet und eingedampft. Der Rückstand wird mit Isopropanol angerieben, filtriert und getrocknet. Man erhält 14,25 g Rohprodukt, das aus Isopropanol umkristallisiert wird, und gewinnt so 13,10 g (71,94 % d. Th.) 1-p-Methoxybenzoyl-2-(2,6-dichlorphenylimino)-imidazolidin vom Fp. 154—156°C.

Beispiel 2

3,37 g (15 mmol) 1-(p-Chlorbenzoyl)-imidazolidin-2-on, 2,43 g (15 mmol) 2,6-Dichloranilin und 50 ml Phosphoroxychlorid werden 1,5 Stunden am Rückfluß erhitzt und anschließend eingedampft. Der Trockenrückstand wird in 30 ml Methylenchlorid digeriert, abgetrennt, mit wenig Methylenchlorid gewaschen und getrocknet. Das so erhaltene Phosphoroxychloridaddukt wird mit 70 ml Eiswasser verrührt und langsam mit 10 %iger Kalilauge versetzt, bis ein konstanter pH von ca. 8 erreicht wird. Der Rückstand wird abfiltriert, mit Wasser gewaschen und getrocknet. Man erhält 2,70 g (48,43 % d. Th.) 1-p-Chlorbenzoyl-2-(2,6-dichlorphenylimino)-imidazolin als analysenreine Substanz, welche ohne weitere Reinigung zur Verarbeitung für pharmazeutische zwecke geeignet ist. Der Fp. beträgt 173—175°C.

Beispiel 3

2,15 g (10 mmol) 1-(p-Cyanobenzoyl)-imidazolidin-2-on, 1,62 g (10 mmol) 2,6-Dichloranilin und 100 ml Phosphoroxychlorid werden 14 Tage bei Raumtemperatur gerührt. Anschließend wird eingedampft und der Rückstand mit 20 ml Methylenchlorid zur Kristallisation gebracht, filtriert, mit wenig Methylenchlorid gewaschen und getrocknet. Das entstandene Phosphoroxychloridaddukt wird in ca. 150 ml Eiswasser suspendiert und langsam mit verdünntem Ammoniak so lange versetzt, bis ein konstanter pH von 8 erreicht ist. Der Rückstand wird abfiltriert, mit Wasser gewaschen und getrocknet. Man erhält so 3,15 g (87,69 % d. Th.) analysenreines 1-(p-Cyanobenzoyl)-2-(2,6-dichlorphenylimino)-imidazolidin vom Fp. 208—210°C. Dieses Produkt kann ohne weitere Reinigung zur Herstellung von pharmazeutischen Produkten verwendet werden.

Beispiel 4

6,24 g (30 mmol) 1-(p-Fluorbenzoyl)-imidazolidin-2-on, 4,86 g (30 mmol) 2,5-Dichloranilin und 18,3 ml (200 mmol) Phosphoroxychlorid werden in 50 ml wasserfreiem Methylenchlorid 70 Stunden am Rückfluß gekocht. Das Lösungsmittel und überschüssiges Phosphoroxychlorid werden unter vermindertem Druck am Rotationsverdampfer abgezogen. Der Rückstand wird, wie in Beispiel 2 angegeben, aufgearbeitet. Das nach dem Eindampfen der Methylenchloridphasen erhaltene Rohprodukt wird aus Acetonitril umkristallisiert. Man erhält so 3,39 g (32,08 % d. Th.) 1-p-Fluorbenzoyl-2-(2,5-dichlorphenylimino-imidazolidin vom Fp. 157—158°C.

Beispiel 5

8,10 g (50 mmol) 2,6-Dichloranilin und 15,4 g (55 mmol) 1-(3,4,5-Trimethoxybenzoylimidazolidin-2-on werden mit 140 ml Phosphoroxychlorid 72 Stunden bei 50°C gerührt. Anschließend wird eingedampft und der Rückstand wird mit Methylenchlorid versetzt, wobei Kristallisation eintritt. Das Kristallisat wird abfiltriert, mit Methylenchlorid gewaschen und getrocknet. Man erhält so 23,9 g Phosphoroxychloridaddukt, welches in 200 ml Wasser suspendiert und mit gesättigter Sodalösung bis zu einem konstanten pH von 7,5 versetzt wird. Dann wird filtriert, mit Wasser gewaschen und aus Isopropanol umkristallisiert. Man erhält so 10,9 g (61,3 % d. Th.) 1-(3,4,5-Trimethoxybenzoyl)-2-(2,6-dichlorphenylimino)-imidazolidin vom Fp. 177-179°C.

Beispiel 6

3,24 g (20 mmol) 2,6-Dichloranilin und 5,17 g (22 mmol) 1-p-Nitrobenzoylimidazolidin-2-on werden in 40 ml Phosphoroxychlorid 120 Stunden bei 80°C gerührt. Nach Beendigung der Reaktion wird das überschüssige Phosphoroxychlorid um Vakuum entfernt, der Rückstand in Methylenchlorid aufgenommen und unter Eiskühlung bis zur vollständigen Neutralisation mit gesättigter Natriumbicarbonsatlösung versetzt. Die Methylenchloridphase wird abgetrennt, die wässerige Phase noch dreimal mit Methylenchlorid extrahiert, die vereinigten Methylenchloridphasen mit Wasser neutral gewaschen, über Natriumsulfat getrocknet und eingedampft. Der Rückstand wird durch Anreiben in Isopropanol zur Kristallisation gebracht, abgetrennt und nacheinander aus Acetonitril und Xylol umkristallisiert. Man erhält so 3,12 g (37,4 % d. Th.) 1-p-Nitrobenzoyl-2-(2,6-dichlorphenylimino)-imidazolidin vom Fp. 195—198°C.

Beispiel 7

3,98 g (22 mmol) 3-Methyl-4-nitrobenzoesäure werden in 75 ml wasserfreiem Tetrahydrofuran gelöst, 3,65 g (22 mmol) N,N'-Carbonyldiimidazol zugesetzt und eine Stunde bei Raumtemperatur gerührt. Zu dieser Lösung tropft man unter Ruhren 4,60 g (20 mmol) 2-(2,6-Dichlorphenylimino)-imidazolidin, welches vorher in 100 ml absolutem Tetrahydrofuran gelöst wurde, und läßt 20 Stunden bei Raumtemperatur stehen. Die Reaktionslösung wird im Vakuum eingedampft, der Rückstand mit Wasser versetzt und dann dreimal mit Äthylacetat extrahiert. Die vereinigten organischen Phasen werden mit Wasser gewaschen,

über Natriumsulfat getrocknet, filtriert und eingedampft. Der Rückstand wird aus Cyclohexan:Isopropanol (1:3) über Aktivkohle umkristallisiert. Man erhält so 5,20 g (66,1 % der Therorie) 1-(3-Methyl-4-nitrobenzoyl)-2-(2,6-dichlorphenylimino)-imidazolidin mit dem Fp. von 152—154°C.

Beispiel 8

4,40 g (22 mmol) p-Methylsulfonyl-benzoesäure werden in 70 ml wasserfreiem Tetrahydrofuran gelöst, 3,56 g (22 mmol) N,N'-Carbonyldiimidazol zugesetzt und eine Stunde bei Raumtemperatur gerührt. Unter weiterem Rühren gibt man eine Lösung von 4,60 g (20 mmol) 2-(2,6-Dichlorphenylimino)-imidazolidin in 50 ml wasserfreiem Tetrahydrofuran tropfenweise zu und läßt 20 Stunden bei Raumtemperatur stehen. Der Eindampfrückstand wird mit Wasser verrieben, abgetrennt, mit Wasser gewaschen, getrocknet und aus Dimethylformamid/Wasser (1:1) umkristallisiert. Man erhält 5,15 g (62,5 % d. Th.) 1-(p-Methylsulfonylbenzoyl)-2-(2,6-dichlorphenylimino)-imidazolidin vom Fp. 240—244°C.

Beispiel 9

3,48 g (21 mmol) Äthoxybenzoesäure werden in 100 ml wasserfreiem Benzol suspendiert, 3,40 g (21 mmol) N,N'-Carbonyldiimidazol zugefügt und eine Stunde bei Raumtemperatur gerührt. Zur klaren Lösung werden 4,60 g (20 mmol) 2-(2,6-Dichlorphenylimino)-imidazolidin zugefügt und 4 Stunden unter Rückfluß gekocht. Nach dem Eindampfen wird der Rückstand mit 100 ml Wasser verrieben, filtriert, mit Wasser gewaschen, getrocknet und aus Toluol umkristallisiert. Man erhält 5,90 g (78 % d. Th.) 1-p-Äthoxybenzoyl-2-(2,6-dichlorphenylimino)-imidazolidin vom Fp. 146—148°C.

Beispiel 10

2,88 g (16 mmol) p-Isopropoxybenzoesäure werden in 30 ml wasserfreiem Dioxan gelöst, 2,59 g 16 mmol N,N'-Carbonyldiimidazol zugefügt und 3 Stunden bei Raumtemperatur belassen. Dazu wird eine Lösung von 3,45 g (15 mmol) 2-(2,6-Dichlorphenylimino)-imidazolidin gegeben und 2 Stunden am Rückfluß erhitzt. Es wird wie in Beispiel 7 aufgearbeitet und aus Benzol/Cyclohexan (1:1) umkristallisiert.

Man erhält so 4,45 g (75,7 % d. Th.) 1-p-Isopropoxybenzoyl-2-(2,6-dichlorphenylimino-imidazolidin von Fp. 146—148°C.

Beispiel 11

1,74 g (11 mmol) p-Fluorbenzoylchlorid werden in 50 ml wasserfreiem Tetrahydrofuran gelöst, 1,50 g (22 mmol) Imidazol zugefügt und 1 Stunde am Rückfluß gekocht. Nach dem Abkühlen auf Raumtemperatur wird vom ausgefallenen Imidazolyhydrochlorid abfiltriert, der Filterrückstand mit wasserfreiem Tetrahydrofuran gewaschen, die vereinigten Filtrate mit 2,10 g (10 mmol) 2-(2-Chlor-6-methylphenylimino)-imidazolidin versetzt und 6 Stunden am Rückfluß erhitzt. Zur Aufarbeitung wird das Lösungsmittel unter vermindertem Druck abgezogen und der Rückstand mit Wasser digeriert, wobei Kristallisation eintritt. Es wird filtriert, mit Wasser gewaschen und getrocknet. Nach dem Umkristallisieren aus Isopropanol erhält man 2,75 g (74,3 % d. Th.) 1-p-Fluorbenzoyl-2-(2-chlor-6-methyl-phenylimino)-imidazolidin vom Fp. 143—145°C.

Beispiel 12

3,31 g (48 mmol) 1,2,4-Triazol werden in 80 ml wasserfreiem Tetrahydrofuran gelöst, mit 4,80 g (24 mmol) 3,4-Dimethoxybenzoylchlorid versetzt und über Nacht bei Raumtemperatur gerührt. Zur Vervollständigung der Reaktion wird noch 2 Stunden am Rückfluß erhitzt. Nach dem Abkühlen wird vom ausgefallenen 1,2,4-Triazolhydrochlorid abfiltriert, der Filterrückstand mit wasserfreiem Tetrahydrofuran gewaschen, die vereinigten Filtrate mit einer Lösung von 4,60 g 2-(2,6-Dichlorphenylimino)-imidazolidin in 50 ml wasserfreiem Tetrahydrofuran versetzt und 16 Stunden bei Raumtemperatur belassen.

Anschließend wird eingedampft, der Rückstand mit Wasser und etwas Natriumbicarbonatlösung verrieben, filtriert, getrocknet und aus Acetonitril umkristallisiert. Man erhält 4,61 g (58,46 % d. Th.) 1-(3,4-Dimethoxybenzoyl)-2-(2,6-dichlorphenylimino)-imidazolidin vom Fp. 201—204°C.

Beispiel 13

2,16 g (11 mmol) 1-(p-Cyanobenzoyl)-imidazol und 2,10 g (10 mmol) 2-(2-Methyl-3-chlorphenylimino)-imidazolidin werden in 50 ml wasserfreiem Tetrachlorkohlenstoff 4 Stunden am Rückfluß erhitzt. Anschließend wird zur Trockne eingedampft under der Rückstand mit 50 ml Wasser verrieben, filtriert, mit Wasser gewaschen, getrocknet und aus Acetonitril umkristallisiert.

Man erhält 2,64 g (78,1 % d. Th.) 1-p-Cyanobenzoyl-2-(2-methyl-3-chlorphenylimino)-imidazolidin vom Fp. 224—228°C.

Beispiel 14

2,26 g (11 mmol) 1-(p-Chlorbenzoyl)-imidazol und 1,89 g (10 mmol) 2-(2,6-Dimethylphenylimino)-imidazolidin werden in 50 ml wasserfreiem Aceton gelöst, 16 Stunden bei Raumtemperatur belassen und wie in Beispiel 12 aufgearbeitet. Nach dem Umkristallisieren aus Isopropanol/Cyclohexan 1:15 erhält man 2,50 g (76,6 % d. Th.) 1-p-Chlorbenzoyl-2-(2,6-dimethylphenylimino)-imidazolidin vom Fp. 143—145°C.

Beispiel 15

1,89 g (5 mmol) 2-[N-(3-Methoxy-4-methylbenzoyl)-N-(2,6-dichlorophenyl)-amino]-imidazolin-2- und 115 mg (0,5 mmol) 2-(2,6-dichlorphenylimino)-imidazolidin werden in 30 ml wasserfreiem Xylol 21 Stunden am Rückfluß gehalten. Nach dem Abkühlen auf Raumtemperatur und mehrstündigem Stehen in der Kälte kristallisieren 1,17 g (61,9 % d. Th.) reines 1-(3-Methoxy-4-methylbenzoyl)-2-(2,6-dichlorphenylimino)-imidazolidin vom Fp. 179—182°C aus.

Beispiel 16

3,96 g (20 mmol) N-p-Chlorbenzoyläthylendiamin werden in 7 ml 4 n Natronlauge gelöst und unter Eiskühlung und heftigem Rühren innerhalb von 15 Minuten zu einer Lösung von 4,86 2,3-Dichlorphenylisocyaniddichlorid in 15 ml wasserfreiem Dioxan getropft. Danach wird der Ansatz auf 50°C erwärmt und 2 Stunden bei dieser Temperatur belassen. Nach dem Abdekantieren der Mutterlauge wird das entstandene Kristallisat mit Wasser verrührt, filtriert, mit Wasser gewaschen und getrocknet. Anschließend wird aus Acetonitril unter Zusatz von Aktivkohle umkristallisiert, wobei man 3,59 g (48,7 % d. Th.) 1-p-chlorbenzoyl-2-(2,3-dichlorphenylimino)-imidazolidin vom Fp. 196—197°C erhält.

Nach dem in den Beispielen 1—16 beschriebenen Verfahren wurden folgende Verbindungen erhalten:

Beispiel 17

1-m-Fluorbenzoyl-2-(2,6-dichlorphenylimino)-imidazolidin (83,48 % d. Th. aus Acetonitril), Fp. 177—179°C.

Beispiel 18

1-p-Fluorbenzoyl-2-(2,6-dichlorphenylimino)-imidazolidin (78,93% d. Th. aus Acetonitril), Fp. 169—171°C.

Beispiel 19

1-(3,4-Dimethoxybenzoyl)-2-(2-chlor-6-methylphenylimino)-imidazolidin (32,55 % d. Th. aus Isopropanol), Fp. 155—157°C.

Beispiel 20

1-p-Methoxybenzoyl-2-(2-Chlor-4-methylphenylimino)-imidazolidin (34,81 % d. Th. aus Acetonitril), Fp. 180—182°C.

Beispiel 21

1-(p-n-Propoxybenzoyl)-2-(2,6-dichlorphenylimino)-imidazolidin (28,2 % d. Th. aus Isopropanol), Fp. 134—136°C.

Beispiel 22

1-P-Chlorbenzoyl-2-(2,5-dichlorphenylimino)-imidazolidin (48,83 % d. Th. aus Isopropanol), Fp. 165—167°C.

Beispiel 23

1-p-Cyanobenzoyl-2-(2,6-dibromphenylimino)-imidazolidin (59,21 % d. Th. aus Toluol), Fp. 224—227°C.

Beispiel 24

1-p-Cyanobenzoyl-2-(3,4-dichlorphenylimino)-imidazolidin (16,52 % d. Th. aus Acetonitril), Fp. 173—175°C.

Beispiel 25

1-p-Nitrobenzoyl-2-(2,3-dichlorphenylimino)-imidazolidin (34,8 % d. Th. aus Acetonitril), Fp. 210—211°C.

Beispiel 26

1-p-Fluorbenzoyl-2-(2-methoxyphenylimino)-imidazolidin (24,2 % d. Th. aus Acetonitril), Fp. 220—221°C.

Beispiel 27

1-m-Cyanobenzoyl-2-(2,6-dichlorphenylimino)-imidazolidin (73,71 % d. Th. aus Acetonitril), Fp. 180—182°C.

Beispiel 28

1-p-Cyanobenzoyl-2-(2,5-dichlorphenylimino)-imidazolidin (29,05 % d. Th. aus Acetonitril), Fp. 186—188°C.

Beispiel 29

1-p-Cyanobenzoyl-2-(2-methyl-3-chlorphenylimino)-imidazolidin (38,76 % d. Th. aus Acetonitril), Fp. 224—228°C.

Beispiel 30

1-(p-n-Propoxybenzoyl)-2-(2,3-dichlorphenylimino)-imidazolidin (16,44 % d. Th. aus Cyclohexan), Fp. 110—112°C.

Beispiel 31

1-p-Chlorbenzoyl-2-(2-chlor-6-methylphenylimino)-imidazolidin (66,41 % d. Th. aus Isopropanol), Fp. 154—156°C.

Beispiel 32

1-(3,5-Dimethoxybenzoyl)-2-(2,6-dichlorphenylimino)-imidazolidin (77,4 % d. Th. aus Isopropanol), Fp. 169—170°C.

Beispiel 33

1-p-Methoxybenzoyl-2-(2-chlor-6-methylphenylimino)-imidazolidin (67,5 % d. Th. aus Isopropanol), Fp. 156—157°C.

Beispiel 34

1-p-Äthoxybenzoyl-2-(2-chlor-6-methyl-phenylimino)-imidazolidin (76,99 % d. Th. aus Isopropanol), Fp. 140—142°C.

Beispiel 35

1-p-Äthoxybenzoyl-2-(2,3-dichlorphenylimino)-imidazolidin (88,69 % d. Th. aus Acetonitril/Isopropanol 1:1), Fp. 153—156°C.

Beispiel 36

1-p-Cyanobenzoyl-2-(2,3-dichlorphenylimino)-imidazolidin (89,1 % d. Th. aus Acetonitril), Fp. 225—228°C.

Beispiel 37

1-p-Brombenzoyl-2-(2,6-dichlorphenylimino)-imidazolidin (87,15 % d. Th. aus Isopropanol), Fp. 176—178°C.

Beispiel 38

1-m-Fluorbenzoyl-2-(2,3-dichlorphenylimino)-imidazolidin (66,81 % d. Th. aus Isopropanol), Fp. 141—142°C.

Beispiel 39

1-p-Methylsulfonylbenzoyl-2-(2,3-dichlorphenylimino)-imidazolidin (59,91 % d. Th. aus Acetonitril), Fp. 193—195°C.

Beispiel 40

1-m-Chlorbenzoyl-2-(2,6-dichlorphenylimino)-imidazolidin (83,82 % d. Th. aus Acetonitril), Fp. 152—154°C.

Beispiel 41

1-[p-(1-Methyl-propyloxy)-benzoyl]-2-(2,3-dichlorphenylimino)-imidazolidin (69,1 % d. Th. aus Isopropanol/Wasser), Fp. 85—88°C.

Beispiel 42

1-[p-(1-Methyl-propyloxy)-benzoyl]-2-(2,6-dichlorphenylimino)-imidazolidin (62,2 % d. Th. aus Isopropanol/Wasser), Fp. 137—140°C.

Beispiel 43

1-p-Isopropoxybenzoyl-2-(2,3-dichlorphenylimino)-imidazolidin (83,8 % d. Th. aus Benzol/Cyclohexan 1:1), Fp. 106—109°C.

Beispiel 44

1-p-Methoxybenzoyl-2-(2,3-dichlorphenylimino)-imidazolidin (97,4 % d. Th. aus Toluol), Fp. 150—152°C.

Beispiel 45

1-p-Cyanobenzoyl-2-(2-methyl-5-fluor-phenylimino)-imidazolidin (81,91 % d. Th. aus Toluol), Fp. 169—171°C.

Beispiel 46

1-p-Cyanobenzoyl-2-(3,4-dichlorphenylimino)-imidazolidin (91,59 % d. Th. aus Toluol), Fp. 173—175°C.

Beispiel 47

1-p-Methoxybenzoyl-2-(2,6-dimethylphenylimino)-imidazolidin (84,16 % d. Th. aus Isopropanol,) Fp. 171—174°C.

Beispiel 48

1-p-Fluorbenzoyl-2-(2,3-dichlorphenylimino)-imidazolidin (38,07 % d. Th. aus Acetonitril) Fp. 184—186°C.

Beispiel 49

1-(p-n-Butoxybenzoyl)-2-(2,6-dichlorphenylimino)-imidazolidin vom Fp. 98—100°C.

Beispiel 50

1-p-Methoxybenzoyl-2-(2-chlor-6-methyl-phenylimino)-imidazolidin vom Fp. 156—157°C.

**Patentansprüche**

1. Substituierte 1-Benzoyl-2-phenylimino-imidazolidine der allgemeinen Formel

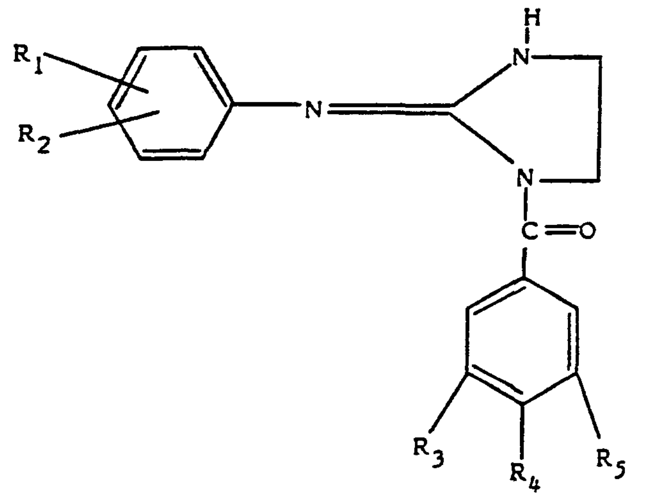

I,

in der

$R_1$ und $R_2$ unabhängig voneinander Wasserstoff, Halogen, die Methyl- oder Methoxygruppe bedeuten und

$R_3$, $R_4$ und $R_5$ unabhängig voneinander für Wasserstoff, die Methylgruppe oder für elektronegative Substituenten aus der Gruppe Halogen, geradkettiger oder verzweigter $C_1$ bis $C_4$-Alkoxyrest, Cyano-, Nitro- oder Methylsulfonylrest stehen, mit der Maßgabe, daß eine der Reste $R_3$ bis $R_5$ immer ein elektronegativer Substituent aus der Gruppe Halogen, geradkettiger oder verzweigter $C_1$ bis $C_4$-Alkoxyrest, Cyano-, Nitro- oder Methylsulfonylrest ist und die beiden anderen Reste unabhängig voneinander die für $R_3$, $R_4$ bzw. $R_5$ angegebene Bedeutung haben, sowie die pharmazeutisch annehmbaren Säureadditionssalze dieser Verbindungen, wobei solche Verbindungen aus der Formel I ausgenommen sind, bei denen $R_1$ 2-Chlor und $R_2$ 4-Chlor bedeutet.

2. Verbindungen gemäß Anspruch 1, in denen $R_1$ und $R_2$ Chlor, Brom, Fluor, die Methyl- oder Methoxygruppe bedeuten.

3. Verbindungen gemäß Anspruch 1, in denen $R_1$ und $R_2$ gleichzeitig Halogen bedeuten und einer der Reste $R_1$ oder $R_2$ in ortho Stellung am Phenylrest steht.

4. Verbindungen gemäß Anspruch 1, in denen $R_1$ 2-Chlor und $R_2$ 6-Chlor, 3-Chlor, 4-Methyl oder 6-Methyl bedeutet.

5. Verbindungen gemäß Anspruch 1, in denen $R_1$ 2-Brom und $R_2$ 6-Brom bedeutet.

6. Verbindungen gemäß Anspruch 1, in denen $R_1$ 2-Methyl und $R_2$ 6-Methyl bedeutet.

7. Verbindungen gemäß Anspruch 1, in denen $R_1$ 2-Methoxy und $R_2$ Wasserstoff bedeutet.

8. Verbindungen gemäß den Ansprüchen 1 bis 7, in denen $R_3$, $R_4$ oder $R_5$ die Cyano-, Nitro-, Methylsulfonylgruppe, einen geradkettigen oder verzweigten $C_1$—$C_4$ Alkoxyrest, Chlor oder Fluor bedeuten.

9. Verbindungen gemäß den Ansprüchen 1 bis 7, in denen einer der Reste $R_3$, $R_4$ oder $R_5$ der Cyano-, Fluor-, Nitro-, Methylsulfonyl-, Chlor- oder Methoxyrest ist und die beiden anderen Reste unabhängig voneinander Wasserstoff oder die Methylkgruppe bedeuten.

10. Verfahren zur Herstellung der Verbindungen der Formel I gemäß Anspruch 1, dadurch gekennzeichnet, daß man

a) ein Anilinderivat der allgemeinen Formel

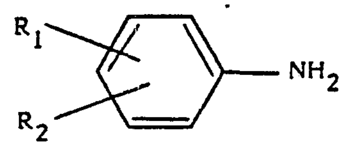

II,

worin $R_1$ und $R_2$ die oben angeführte Bedeutung besitzen, mit einem substituierten 1-Benzoyl-imidazolidin-2-on der allgemeinen Formel

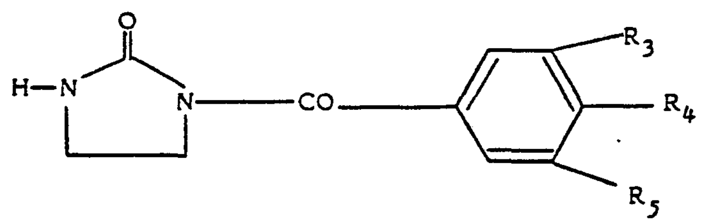

III,

worin $R_3$ bis $R_5$ wie in Formel I definiert sind, im Molverhältnis 1:1 bis 1:1,1 in Gegenwart von mindestens 2 Moläquivalenten Phosphoroxychlorid umsetzt oder

b) ein substituiertes 2-Phenylimino-imidazolidin der allgemeinen Formel

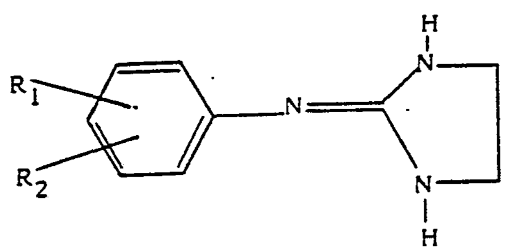

IV,

worin $R_1$ und $R_2$ die oben angeführte Bedeutung besitzen, mit einem aktiven Amid der allgemeinen Formel

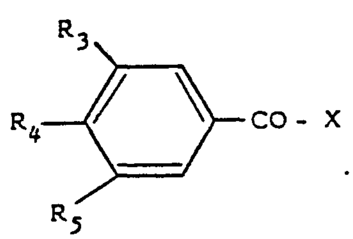

V,

umsetzt, in der $R_3$ bis $R_5$ wie in Formel I definiert sind und X den Rest eines quasiaromatischen fünfgliederigen Heterocyclus mit mindestens 2 Stickstoffatomen im Ring, der gegebenenfalls mit einem Benzolkern kondensiert sein kann, oder den Rest der allgemeinen Formel

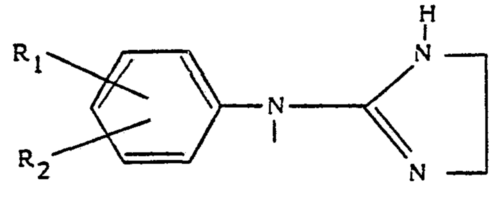

VI,

darstellt, wobei $R_1$ und $R_2$ die in Formel I angegebene Bedeutung besitzen oder

c) ein substituiertes N-Benzoyläthylendiamin der allgemeinen Formel

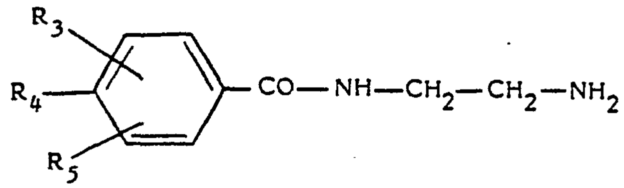

VII,

in der $R_3$, $R_4$ und $R_5$ die oben angegebene Bedeutung besitzen, mit einem Isocyaniddichlorid der allgemeinen Formel

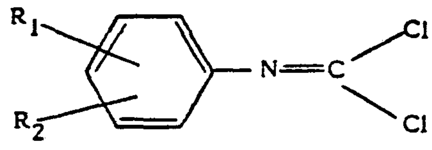

VIII,

in der $R_1$ und $R_2$ die oben angegebene Bedeutung besitzen, in Gegenwart von 2 Moläquivalenten eines Alkalihydroxids oder Alkalihydrids als säurebindendes Mittel umsetzt und die so erhaltenen Verbindungen gegebenenfalls mit Säuren in ihre pharmazeutisch annehmbaren Salze überführt.

11. Arzneimittel, dadurch gekennzeichnet, daß es als Wirkstoff eine oder mehrere Verbindungen der allgemeinen Formel I oder deren pharmazeutisch akzeptablen Säureadditionssalze gemäß Anspruch 1 enthält.

12. Verfahren zur Herstellung von Arzneimitteln gemäß Anspruch 11, dadurch gekennzeichnet, daß man eine oder mehrere Verbindungen der allgemeinen Formel I oder deren Säureadditionssalze mit den üblichen galenischen Hilfs-, Träger-, Spreng- oder Schmiermitteln oder Substanzen zur Erzielung einer Depotwirkung zu Tabletten, Kapseln, Dragees, Zäpfchen, Pulvern, Lösungen oder Suspensionen formuliert.

**Revendications**

1. 1-Benzoyl-2-phénylimino-imidazolidines substituées de formule générale:

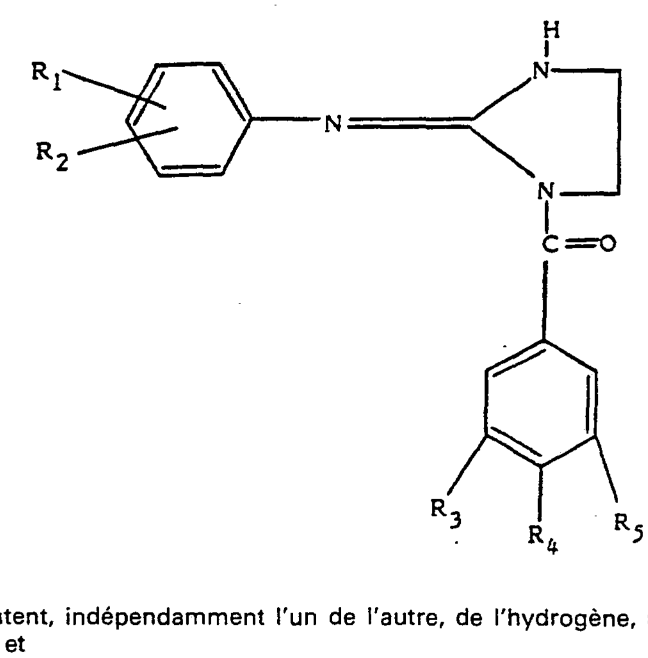

I,

dans laquelle:

$R_1$ et $R_2$ représentent, indépendamment l'un de l'autre, de l'hydrogène, un halogène ou le groupe méthyle ou méthoxy, et

$R_3$, $R_4$ et $R_5$ représentent, indépendamment l'un de l'autre de l'hydrogène, le groupe méthyle ou des substituants électronégatifs du groupe halogène, radical alcoxy en $C_1$ à $C_4$ linéaire ou ramifié, radical cyano, nitro ou méthylsulfonyle, à la condition que l'un des radicaux $R_3$ à $R_5$ soit toujours un substituant électronégatif du groupe halogène, radical alcoxy en $C_1$ à $C_4$ linéaire ou ramifié, radical cyano, nitro ou méthylsulfonyle et les deux autres radicaux ont indépendamment l'un de l'autre, la signification indiquée pour $R_3$, $R_4$ ou $R_5$, ainsi que les sels d'addition avec des acides de ces composés pharmaceutiquement acceptables, à l'exclusion des composés de formule I dans lesquels $R_1$ est un substituant 2-chloro et $R_2$ un substituant 4-chloro.

2. Composés suivant la revendication 1, dans lesquels $R_1$ et $R_2$ représentent du chlore, du brome, du fluor ou le groupe méthyle ou méthoxy.

3. Composés suivant la revendication 1, dans lesquels $R_1$ et $R_2$ représentent, simultanément, un halogène et l'un des radicaux $R_1$ ou $R_2$ est position ortho sur le radical phényle.

4. Composés suivant la revendication 1, dans lesquels $R_1$ est un substituant 2-chloro et $R_2$ est un substituant 6-chloro, 3-chloro, 4-méthyle ou 6-méthyle.

5. Composés suivant la revendication 1, dans lesquels $R_1$ est un substituant 2-bromo et $R_2$ est un substituant 6-bromo.

6. Composés suivant la revendication 1, dans lesquels $R_1$ est un substituant 2-méthyle et $R_2$ est un substituant 6-méthyle.

7. Composés suivant la revendication 1, dans lesquels $R_1$ est un substituant 2-méthoxy et $R_2$ est de l'hydrogène.

8. Composés suivant l'une quelconque des revendications 1 à 7, dans lesquels $R_3$, $R_4$ ou $R_5$ représente le groupe cyano, nitro ou méthylsulfonyle, un radical alcoxy en $C_1$—$C_4$ linéaire ou ramifié, du chlore ou du fluor.

9. Composés suivant l'une quelconque des revendications 1 à 7, dans lesquels l'un des radicaux $R_3$, $R_4$ ou $R_5$ est le radical cyano, fluoro, nitro, méthylsulfonyle, chloro ou méthoxy et les deux autres radicaux représentent, indépendamment l'un de l'autre, de l'hydrogène ou le groupe méthyle.

10. Procédé pour la préparation des composés de formule I suivant la revendication 1, caractérisé en ce que

a) on fait réagir un dérivé d'aniline de formule générale

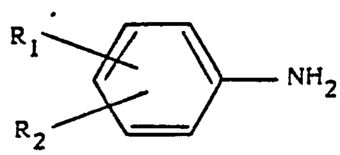
II,

dans laquelle $R_1$ et $R_2$ ont la signification indiquée ci-dessus avec une l-benzoyl-imidazolidin-2-one substituée de formule générale

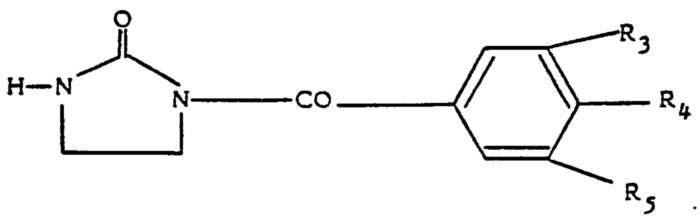
III,

dans laquelle $R_3$ à $R_5$ sont tels que définis dans la formule I, dans un rapport molaire de 1:1 à 1:1,1, en présence d'au moins deux équivalents molaires d'oxychlorure de phosphore, ou

b) on fait réagir une 2-phénylimino-imidazolidine substituée de formule générale

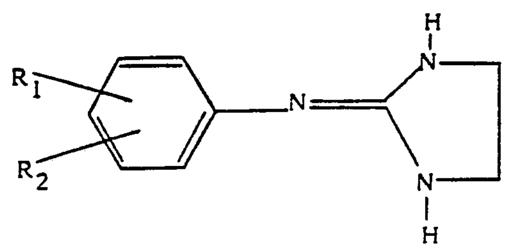
IV,

dans laquelle $R_1$ et $R_2$ ont la signification indiquée ci-dessus, avec un amide actif de formule générale

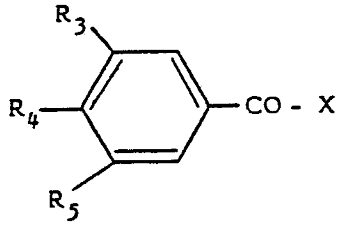
V,

dans laquelle $R_3$ à $R_5$ sont tels que définis dans la formule I et X est le radical d'un hétérocycle à cinq chaînons quasiaromatique comprenant au moins 2 atomes d'azote dans le cycle, qui éventuellement peut

être condensé avec un noyau benzène, ou le radical de formule générale

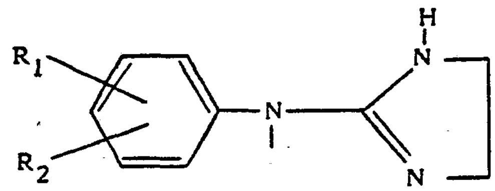

VI,

dans laquelle $R_1$ et $R_2$ ont la signification indiquée dans la formule I, ou

c) on fait réagir une N-benzoyléthylènediamine substituée de formule générale

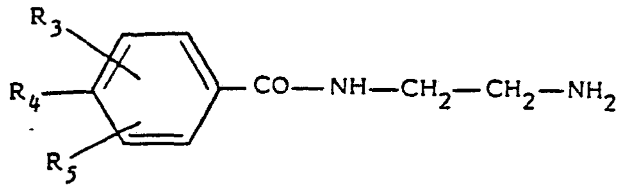

VII,

dans laquelle $R_3$, $R_4$ et $R_5$ ont la signification indiquée ci-dessus avec un isocyanure-dichlorure de formule générale

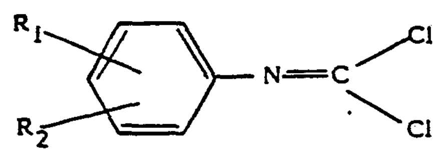

VIII,

dans laquelle $R_1$ et $R_2$ ont la signification indiquée ci-dessus, en présence de 2 équivalents molaires d'un hydroxyde alcalin ou d'un hydrure alcalin àtitre d'agent fixateur d'acide,
et on convertit éventuellement les composés ainsi obtenus en leurs sels pharmaceutiquement acceptables avec des acides.

11. Médicament, caractérisé en ce qu'il contient, à titre de substance active, un ou plusieurs composés de formule générale I ou leurs sels d'addition avec des acides pharmaceutiquement acceptables suivant la revendication 1.

12. Procédé pour la préparation de médicaments suivant la revendication 11, caractérisé en ce qu'on formule un ou plusieurs composés de formule générale I ou leurs sels d'addition avec des acides avec les agents adjuvants, formant véhicules ou supports, de dispersion ou lubrifiants galéniques usuels, ou avec des substances pour obtenir un effet retard, en comprimés, capsules ou gélules, dragées, suppositoires, poudres, solutions ou suspensions.

**Claims**

1. Substituted 1-benzoyl-2-phenylimino-imidazolidines of the general formula

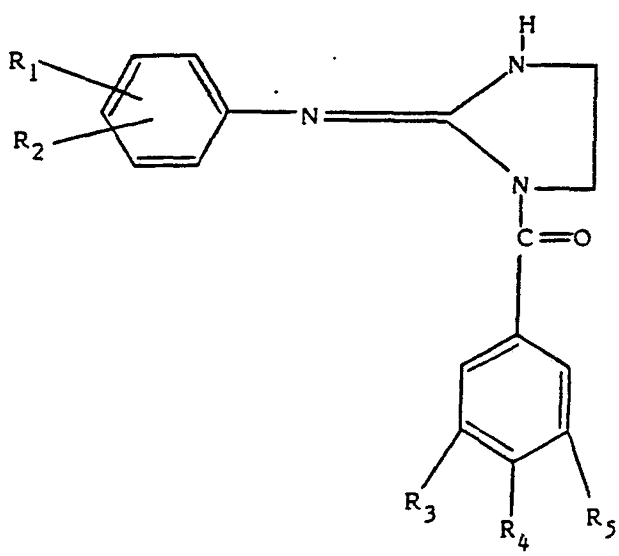

I,

in which

$R_1$ and $R_2$ independently of one another denote hydrogen, halogen, the methyl group or the methoxy group and

$R_3$, $R_4$ and $R_5$ independently of one another represent hydrogen, the methyl group or electronegative substituents from the group of halogen, straight-chain or branched $C_1$ to $C_4$-alkoxy radical, cyano, nitro or methylsulphonyl radical, with the proviso that one of the radicals $R_3$ to $R_5$ is always an electronegative substituent from the group of halogen, straight-chain or branched $C_1$ to $C_4$-alkoxy radical, cyano, nitro or methylsulphonyl radical and the two other radicals independently of one another have the meaning given for $R_3$, $R_4$ or $R_5$,

as well as the pharmaceutically acceptable acid addition salts of these compounds, with the exception of those compounds of the formula I in which $R_1$ denotes 2-chloro and $R_2$ denotes 4-chloro.

2. Compounds according to Claim 1, in which $R_1$ and $R_2$ denote chlorine, bromine, fluorine, the methyl group or the methoxy group.

3. Compounds according to Claim 1, in which $R_1$ and $R_2$ simultaneously denote halogen and one of the radicals $R_1$ or $R_2$ is in the ortho-position on the phenyl radical.

4. Compounds according to Claim 1, in which $R_1$ denotes 2-chloro and $R_2$ denotes 6-chloro, 3-chloro, 4-methyl or 6-methyl.

5. Compounds according to Claim 1, in which $R_1$ denotes 2-bromo and $R_2$ denotes 6-bromo.

6. Compounds according to Claim 1, in which $R_1$ denotes 2-methyl and $R_2$ denotes 6-methyl.

7. Compounds according to Claim 1, in which $R_1$ denotes 2-methoxy and $R_2$ denotes hydrogen.

8. Compounds according to Claims 1 to 7, in which $R_3$, $R_4$ or $R_5$ denote the cyano, nitro or methylsulphonyl group, a straight-chain or branched $C_1$-$C_4$-alkoxy radical, chlorine or fluorine.

9. Compounds according to Claims 1 to 7, in which one of the radicals $R_3$, $R_4$ or $R_5$ is the cyano, fluorine, nitro, methylsulphonyl, chlorine or methoxy radical and the other two radicals independently of one another denote hydrogen or the methyl group.

10. Process for the preparation of the compounds of the formula I according to Claim 1, characterised in that

a) an aniline derivative of the general formula

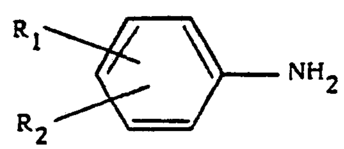

II,

wherein $R_1$ and $R_2$ have the abovementioned meaning, is reacted with a substituted 1-benzoyl-imidazolidin-2-one of the general formula

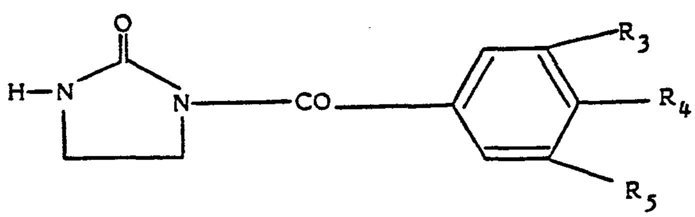

III,

wherein $R_3$ to $R_5$ are defined as in formula I, in the molar ratio of 1:1 to 1:1.1, in the presence of at least two mole equivalents of phosphorus oxychloride or

b) a substituted 2-phenylimino-imidazolidine of the general formula

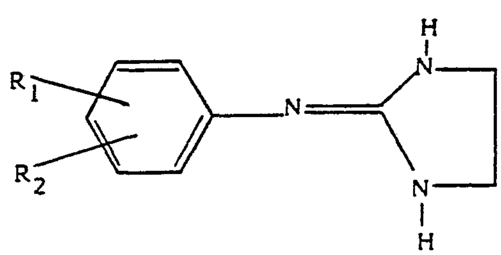

IV,

wherein $R_1$ and $R_2$ have the abovementioned meaning, is reacted with an active amide of the general formula

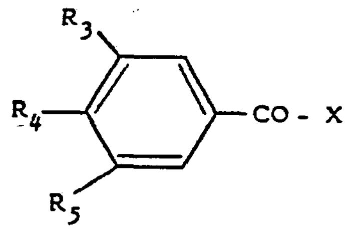

V,

in which $R_3$ to $R_5$ are defined as in formula I and X represents the radical of a quasi-aromatic five-membered heterocyclic structure with at least two nitrogen atoms in the ring, which can optionally be fused to a benzene nucleus, or the radical of the general formula

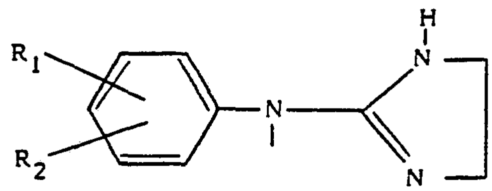

VI,

wherein $R_1$ and $R_2$ have the meaning given in formula 1 or

c) a substituted N-benzoylethylenediamine of the general formula

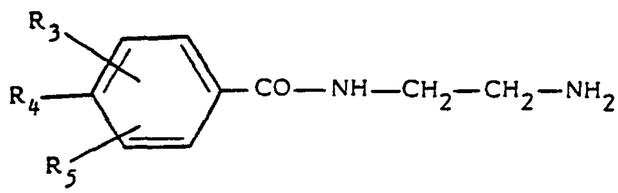

VII,

in which $R_3$, $R_4$ and $R_5$ have the abovementioned meaning, is reacted with an isocyanide-dichloride of the general formula

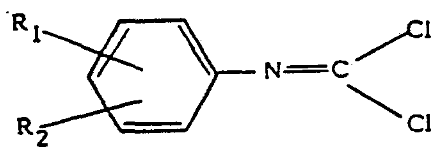

VIII,

in which $R_1$ and $R_2$ have the abovementioned meaning, in the presence of 2 mole equivalents of an alkali metal hydroxide or an alkali metal hydride as an acid-binding agent
and, if desired, the compounds thus obtained are converted by means of acids into their pharmaceutically acceptable salts.

11. Medicament, characterised in that it contains, as the active substance, one or more compounds of the general formula I or their pharmaceutically acceptable acid addition salts according to Claim 1.

12. Process for the preparation of medicaments according to Claim 11, characterised in that one or more compounds of the general formula I or their acid addition salts are formulated with the customary galenical auxiliaries, carriers, disintegrating agents or lubricants or substances for achieving a depot effect, to give tablets, capsules, dragees, suppositories, powders, solutions or suspensions.